Europäisches Patentamt

(19) European Patent Office

Office européen des brevets

(11) Publication number: **0 203 376**
**B1**

# EUROPEAN PATENT SPECIFICATION

(45) Date of publication of the patent specification:
27.12.89

(51) Int. Cl.⁴: **A 61 L  2/02,** B 08 B  3/12,
G 02 C  13/00

(21) Application number: **86105662.0**

(22) Date of filling: **24.04.86**

(54) Miniature cleaning device.

(30) Priority: **24.04.85  US 726742**

(43) Date of publication of application:
**03.12.86 Bulletin 86/49**

(45) Publication of the grant of the patent:
**27.12.89 Bulletin 89/52**

(84) Designated Contracting States:
**CH DE FR GB IT LI**

(56) References cited:
**DE-A-3 106 519**
**FR-A-2 143 252**
**US-A-3 720 402**
**US-A-3 973 760**

(73) Proprietor: **Pilkington Visioncare, Inc. (a Delaware
corp.), 895 Kifer Road, Sunnyvale California (US)**

(72) Inventor: **Bernfeld, Morris, 33- 64 21st Street, Long
Island City New York (US)**
Inventor: **Kamen, Melvin E., 51 Chestnut Ridge
Roads, Woodcliff Lake New Jersey (US)**

(74) Representative: **Schmidt- Evers, Jürgen, Dipl.- Ing.,
Patentanwälte Dipl.- Ing. H. Mitscherlich Dipl.-
Ing. K. Gunschmann Dipl.- Ing. Dr.rer.nat. W.
Körber Dipl.- Ing. J. Schmidt- Evers Dipl.- Ing.
W. Melzer Steinsdorfstrasse 10, D-8000 München
22 (DE)**

## Description

The present invention relates to devices for cleaning small objects such as hard or soft contact lenses, jewelry, and the like. Contact lenses acquire deposits (collectivly known as "soil") of dirt from the athmosphere and organic material exuded by the eye or dissolved in tears. This soil should be removed daily to promote eye hygiene and clear vision.

Devices are known which impart vibrations in the ultrasonic range to fluids in order to clean the surfaces of objects immersed in the fluid. Such devices generally have rigid walls and bottoms, so that they can resonate. Up to now, it has generally been believed that optimizing the performance of such cleaning devices required determining optimal frequency ranges, or adding features such as heaters or special cleaning solutions.

For example, McLure U.S. Patent 9 973 760 and Dieter, et al., DE-A-3 106 519 generally relate to apparatus for cleaning and sterilizating small objects such as soft contact lenses; however, none disclose or suggest the use, in combination with flexible wall or bottom portion, of a vibrator adapted for vibrating said wall in the frequency range of about 2 to about 120 times per second, these references only disclosing transducers vibrating in the ultrasonic range while being attached to rigid wall portions of the cleaning tank.

The present invention comprises a device useful for cleaning the surface of small objects, comprising:

(a) a watertight tank, open at the top, for receiving a liquid into which the small objects to be cleaned are to be immersed;
(b) vibrator means attached to the exterior of a wall or bottom portion of the tank for vibrating said portion;
(c) means for supporting said tank while keeping at least said wall or bottom portion free from contact with structure other than the vibrator means;

characterized in that at least said wall or bottom portion of the tank to which said vibrator means is attached is made of flexible material, and in that said vibrator means is adapted for vibrating said flexible wall or bottom portion at about 2 to 120 times per second.

The present invention will be described in accordance with the following drawings, wherein

Figure 1 is a perspective view of one embodiment of the invention and

Figure 2 is a cross-section view of the embodiment of Figure 1

Figure 3 is a perspective view of a device useful for holding objects such as soft contact lenses in the device of the present invention and

Figure 4 is a cross-section of the embodiment of Figure 2 including a different device for holding objects to be cleaned.

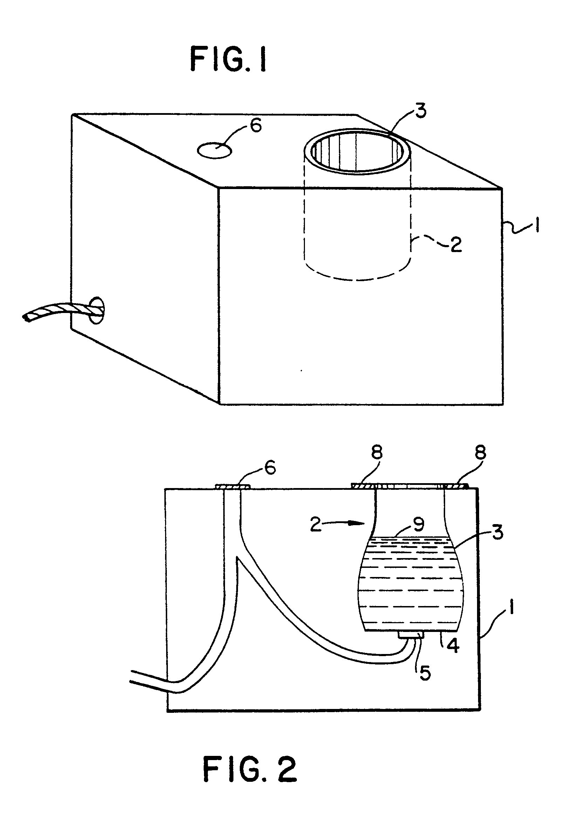

Referring to Figure 1 and 2, the invention includes a base 1 of rigid material which supports the tank 2. Base 1 can be provided in any of a large variety of versions, such as a small hollow box or simply a set of legs. In a preferred embodiment, tank 2 hangs freely from or within base 1. Tank 2 can be glued in place to hand from an opening in the top surface of base 1, formed with a rigid collar 8 that rests on the top surface of base 1. The base 1 should support tank 2 in a manner such that no structure other than vibrating means 5 contacts the exterior of the flexible surface of tank 2 which are described below.

Tank 2 generally includes sidewalls 3 and a bottom 4. The tank 2 can be molded from one continuous piece of material, to avoid seams which could develop leaks. Alternatively, the tank can also be assembled from separate pieces of material. It will be appreciated that the sidewalls can be distinct planar sections, or there can be one continuously curving sidewall having a generaly circular or elliptical horizontal cross-section.

At least one surface, i.e., sidewall or bottom, should be made of flexible material which is capable of transmitting vibrations from the vibrating means 5 to liquid which is held inside the tank. The other surfaces can also be made of flexible material, or can be made of rigid material such rigid poiyethylene, aluminum, or the like. Examples of satisfactory flexible material include natural rubber or synthetic rubber such as butyl rubber, styrenebutadiene rubber, or synthetic polymeric material such as polyethlene, nylon, polypropylene, polyvinyl chloride, polyurethane or silicone rubber. The flexible material should be on the order of about 2 to about 50 about 50.8 to about 1270 µm mils thick. Preferably, the flexible material should have good dimensional stability, by which is meant that if it is pulled or stretched from its normal position and then released it reassumes its normal position.

Vibrating means 5 is positioned so that it contacts the flexible surface, so that when means 5 is activated its vibrations are transmitted to fluid inside the tank. The vibrating means 5 can be glued to the flexible surface if the tank is not remoavble. It can be an ordinary buzzer or vibrator or solenoid, or any other device (preferably electrically actuated) which vibrates at a rate of about 2 to about 120 times per second, and preferably about 20 to about 120 times per second. Conveniently, the vibrator can be coupled to household current so that it vibrates at the frequency of that current, e.g., about 60 cycles per second. Vibrating means 5 is connected via switch to a source of alternating current or direct current, such as a plug for connection with ordinary household current or a battery contained within base 1. Advantageously, provision can be included in the circuitry so that the frequency at which means 5 vibrates can be varied. The vibration creates a standing wave in the liquid.

The invention also advantageously includes a support means for use when the object being cleaned is less dense than the liquid in the tank. One such object is a "soft", i.e., hydrophilic, contact lens.

The support means should be rigid and should have openings sufficient in number and size so that the vibrations that have been imparted to the liquid can reach the lens or other object being cleaned. Since the support means should envelope the lenses completely, while providing the communication with the liquid, the openings should not be so large that the lens or other object being cleaned can pass through. In this way, the lenses are protected from being damaged by being tossed about within the vibrating liquid. The support means can be made of nylon, polypropylene, or any other equivalent inert, rigid material. The space within which the lens is enveloped should not exceed about twice the lens diameter in any direction.

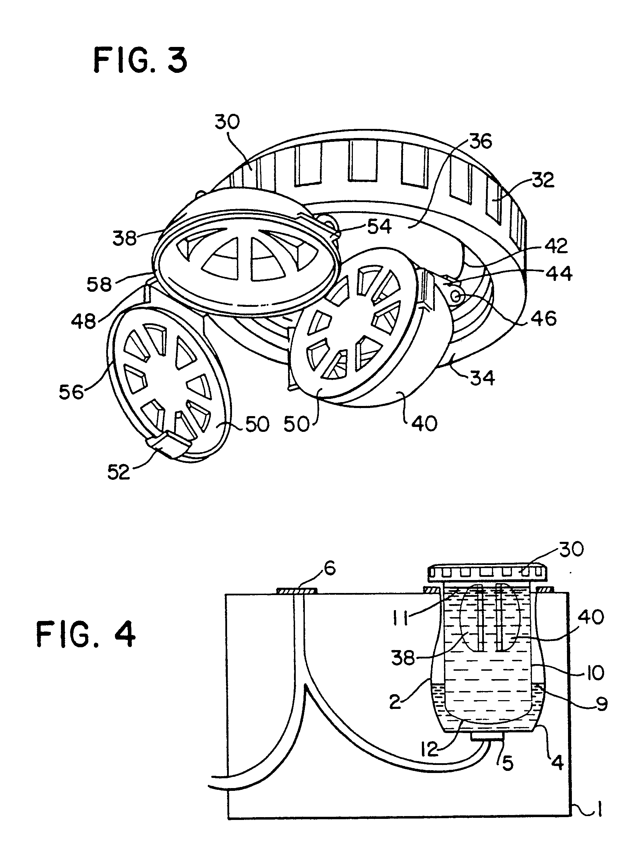

One embodiment of support means is disclosed in U.S. Patent No. 3 997 049 and shown in Figure 3. It comprises a structure including a cap-like housing optionally ringed with notches 32 to make gripping the device easier. Cap 30 includes a lowermost planar surface 34 adapted to fit flush against the top surface of base 1 (see Figure 1). Disposed within cap 30, or optionally at the same level as surface 34, is support plate 36 which includes means for pivotally mounting basket-like lens receptacles 38 and 40. Such mounting means can comprise an angled tab 42 protruding from plate 36, a tab such as tabs 44 extending from receptacle 40, and a pin 46 connecting tabs 42 and 44. Each receptacle should be able to pivot between the two positions in which receptacles 38 and 40 are shown in Figure 3. Referring, for example, to receptacle 38, each receptacle includes a base portion connected via strap hinge 48 to a lid 50. When the lid is closed, flexible arm 52 is snapped over abutment 54 on the base to hold the lid in the closed position. In addition, flange 56 on the lid tightly surrounds upstanding lip 58 on the base, thereby preventing the lens from sliding between the lid and the base. To use this device, each receptacle is rotated into the position occupied by receptacle 38 in Figure 3, the lids are opened, a lens is placed into each receptacle, the lids are snapped shut, and each receptacle is rotated into the position depicted for receptacle 40. The openings shown in both lids and both bases permit liquid to reach both surfaces of both lenses without letting the lenses escape.

To use the invention, the object being cleaned is placed into the tank 2, or placed in a support means, such as the one shown in Figure 3, which turn is placed in tank 2 so that the object(s) will be submerged. It should be understood that the object or the support means can rest on the bottom of tank 2, or can be suspended therein in any known manner. Tank 2 is then filled with enough water or another liquid which is capable of trans-

mitting vibrational energy to the object and which is inert to the object. The water should reach a level 9 which submerges the object(s) being cleaned and is higher than the point at which vibration means 5 is attached to the tank. For this reason, it will be evident that vibrating means 5 should be attached at a low position on a sidewall 3, or even to bottom 4 where such placement is feasible. When the object being cleaned is a soft (hydrophilic) contact lens, the water should include enough physiologically inert salts and buffering agents to be isotonic with eye fluids. Generally, sodium chloride and sodium borate/boric acid are used in amounts to provide a pH of 6.5 - 8.5 (preferably 7.0 - 7.9) and osmolarity of 200 - 450 milliosmol per kg of solution. If desired, small amounts (less than 5 wt.%) of a surfactant, a preservative (e.g., chlorhexidine or thimerosal), and/or a sterilizing agent (e.g., hydrogen peroxide) can also be added to the water. Indeed, any commercially available contact lens storage solution can be used in this invention quite satisfactorily, such as those sold as "Soft care" or "Soft Mate" by Barnes-Hind, Inc. of Sunnyvale, California. The particular solutions are well within the skill of the art and are not essential to the results produced by the present invention. The vibrating means is switched on, and allowed to run until the object is cleaned. In general, a running time of about 10 seconds to about 3 hours will be satisfactory for contact lenses. A timer can be incorporated into the switch to provide a measured period of time during which the vibrating means is activated.

Another embodiment for using the invention is shown in Figure 4. Cap 30, or equivalent support means, is placed on an open-topped removable container 10 positioned within tank 2 so that the objects held by that support means are submerged in liquid which is at a level 11 that is above those objects. The liquid in container 10 can be water, preferably, and when the objects are soft contact lenses the water should also have a physiologically acceptable pH and isotonicity as discussed above. The container 10 should have a bottom 11 which is made of flexible material of the type described above. The sides of container 10 can also be flexible, or can be rigid metal or plastic such as polymethyl methacrylate. Bottom 4 of tank 2 should also be flexible, and vibrator 5 should be attached to bottom 4. Vibrator 5 is still considered operably coupled to the liquid surrounding the lenses so long as vibrations of vibrator 5 are transmitted to the surfaces of the lenses in the liquid within container 10. The space between bottoms 4 and 11 can contain liquid, or this space can contain air, or a vacuum, provided that sides 2 and 10 are close enough together that an effectively airtight seal is formed. Instead, bottom 11 can be formed so that is conforms to, and contacts, bottom 4, whereby the vibrations are transferred by direct contact. This lets the user avoid the already low risk of inadvertent electric shock. Alternatively, liquid such as water can be placed in tank 2, outside container 10, so that the vibrations are transferred from bottom 4 to bottom 12 by that liquid. The removability of the user dump out used solution more easily, and store the cleaned lenses in container 10 in a separate location. The vibrating means establishes an intense barrage of liquid against the surface of the object being cleaned. This intensity is unexpectedly enhanced by the flexible surface of the tank. The resulting faster and more thorough cleaning is a significant advantage of the this invention.

## Claims

1. A device for cleaning small objects, comprising

(a) a watertight tank (2), open at the top, for receiving a liquid into which the small objects to be cleaned are to be immersed;
(b) vibrator means (5) attached to the exterior of a wall or bottom portion (4) of the tank (2) for vibrating said portion (4) ; and
(c) means (8) for supporting said tank (2) while keeping at least said wall or bottom portion (4) free from contact with structure other than said vibrator means (5), characterized in that at least said wall or bottom portion (4) of the tank (2) to which said vibrator means (5) is attached is made of flexible material, and in that said vibrator means (5) is adapted for vibrating said flexible wall or bottom portion (4) at about 2 to about 120 times per second.

2. The device of claim 1, characterized in that said small objects are contact lenses immersed in an aqueous solution.

3. The device of claim 2, characterized in a rigid support means (38, 40) for holding said lenses inside said tank (2) whilst enabling said lenses to be contacted with said aqueous solution held in said tank (2).

4. The device of any one of claims 1 to 3, characterized in that said flexible wall or bottom portion (4) of said tank (2) is made thin, flexible, polymeric material.

5. The device one of claims 1 to 4, characterized in that the whole of said tank (2) is made of thin, flexible material.

6. The device of claims 4 or 5, characterized in that said material is synthetic rubber.

7. The device of any one of claims 1 - 6, characterized by an intermediate watertight container (10) inside the thank (2), open at the top, for receiving a liquid into which the small objects to be immersed said container (10) having a flexible wall or bottom portion (12) which is vibrated by said vibrator means (5) vibrating said flexible wall or bottom portion (4).

## Patentansprüche

1. Vorrichtung zum Reinigen kleiner Objekte, mit

a) einem wasserdichten Tank (2), der an seinem oberen Ende offen ist, zum Aufnehmen einer Flüssigkeit, in die die kleinen Objekte, die zu reinigen sind, einzutauchen sind,
b) einem Vibrator (5), der an dem Äußeren eines Wandungsoder eines Boden-Teils (4) des Tanks (2) zum Versetzen des Teils (4) in Schwingungen angebracht ist, und
c) einem Mittel (8) zum Halten des Tanks (2), während zumindest der Wandungs- oder der Boden-Teil (4) frei von einer Berührung mit einem Bauteil, das nicht der Vibrator (5) ist, frei gehalten wird, dadurch gekennzeichnet,

daß zumindest der Wandungs- oder der Boden-Teil (4) des Tanks (2), an dem der Vibrator (5) angebracht ist, aus einem biegungsfähigen Material hergestellt ist und daß der Vibrator (5) dazu bestimmt ist, den biegungsfähigen Wandungs- oder den biegungsfähigen Boden-Teil (4) ungefähr 2- bis ungefähr 120-mal pro Sekunde schwingen zu lassen.

2. Vorrichtung nach Anspruch 1, dadurch gekennzeichnet, daß die kleinen Objekte Kontaktlinsen sind, die in eine wässerige Lösung eingetaucht werden.

3. Vorrichtung nach Anspruch 2, gekennzeichnet durch starre Tragmittel (38, 40) zum Halten der Linsen innerhalb des Tanks (2), wobei die Linsen in Berührung mit der wässerigen Lösung kommen, die von dem Tank (2) aufgenommen ist.

4. Vorrichtung nach einem der Ansprüche 1 bis 3, dadurch gekennzeichnet, daß der biegungsfähige Wandungs- oder der biegungsfähige Boden-Teil (4) des Tanks (2) aus einem dünnen, biegungsfähigen polymeren Material hergestellt ist.

5. Vorrichtung nach einem der Ansprüche 1 bis 4, dadurch gekennzeichnet, daß die Gesamtheit des Tanks (2) aus einem dünnen, biegungsfähigen Material hergestellt ist.

6. Vorrichtung nach Anspruch 4 oder 5, dadurch gekennzeichnet, daß das Material ein synthetischer Gummi ist.

7. Vorrichtung nach einem der Ansprüche 1 bis 6, gekennzeichnet durch einen innerhalb des Tanks (2) angeordneten wasserdichten, an seinem oberen Ende offenen Zwischen-Behälter (10) zum Aufnehmen einer Flüssigkeit, in die die kleinen Objekte, die zu reinigen sind, einzutauchen sind, welcher Behälter (10) einen biegungsfähigen Wandungs- oder einen biegungsfähigen Boden-Teil (12) hat, der durch den Vibrator (5), welcher den biegungsfähigen Wandungs- oder den biegungsfähigen Boden-Teil (4) in Schwingungen versetzt, in Schwingungen versetzt wird.

# EP 0 203 376 B1

7

## Revendications

1. Dispositif pour nettoyer de petits objets, comprenant
   a) un réservoir étanche (2), ouvert à son sommet, pour recevoir un liquide dans lequel les petits objets à nettoyer doivent être immergés,
   b) un moyen vibrateur (5) fixé à l'extérieur d'une partie de paroi ou de fond (4) du réservoir (2) pour faire vibrer ladite partie (4), et
   c) des moyens (8) pour supporter ledit réservoir (2) tout en empêchant au moins ladite partie de paroi ou de fond (4) d'entrer en contact avec une structure autre que ledit moyen vibrateur (5), caractérisé en ce qu'au moins ladite partie de paroi ou de fond (4) du réservoir (2) à laquelle est fixé ledit moyen vibrateur (5) est faite d'un matériau flexible, et en ce que ledit moyen vibrateur (5) est conçu pour faire vibrer ladite partie de paroi ou de fond flexible (4) à environ 2 à environ 120 fois par seconde.

2. Dispositif selon la revendication 1, caractérisé en ce que lesdits petits objets sont des lentilles de contact immergées dans une solution aqueuse.

3. Dispositif selon la revendication 2, caractérisé par un moyen de support rigide (38, 40) pour maintenir lesdites lentilles à l'intérieur dudit réservoir (2), tout en permettent auxdites lentilles d'être mises en contact avec ladite solution aqueuse contenue dans ledit réservoir (2).

4. Dispositif selon l'une des revendications 1 à 3, caractérisé en ce que ladite partie de paroi ou de fond flexible (4) dudit réservoir (2) est faite d'un matériau polymère mince, flexible.

5. Dispositif selon l'une des revendications 1 à 4, caractérisé en ce que la totalité dudit réservoir (2) est faite un matériau mince flexible.

6. Dispositif selon la revendication 4 ou 5, caractérisé en ce que ledit matériau est du caoutchouc synthétique.

7. Dispositif selon l'une des revendications 1 à 6, caractérisé par un conteneur étanche intermédiaire (10) à l'intérieur du réservoir (2), ouvert à son sommet, pour recevoir un liquide dans lequel les petits objets à nettoyer doivent être immergés, ledit conteneur (10) ayant une partie de paroi ou de fond flexible (12) qui est soumise à des vibrations par ledit moyen vibrateur (5) faisant vibrer ladite partie de paroi ou de fond flexible (4).

5

# FIG. I

# FIG. 2

# FIG. 3

# FIG. 4